# EUROPEAN PATENT APPLICATION

(11) **EP 4 202 060 A1**
(43) Date of publication of application: **28.06.2023**
(21) Application number: 21383223.1
(22) Date of filing: 27.12.2021
(51) Int. Cl.: C12Q 1/6883

(54) **CIRCULATING MIRNAS AS PREDICTIVE BIOMARKERS OF THE RISK OF CARDIAC ISCHEMIA IN PATIENTS WITH CHEST PAIN**

(71) Applicant: Fundación Para la Investigación del Hospital Universitario y Politécnico La Fe de la Comunidad Valenciana, 46026 Valencia (ES); Universitat Politècnica de València, 46022 Valencia (ES)
(72) Inventor: ONTORIA OVIEDO, Imelda, 46026 VALENCIA (ES); PEIRÓ MOLINA, Esteban, 46026 VALENCIA (ES); HERNANDIZ MARTÍNEZ, Amparo, 46026 VALENCIA (ES); IGUAL MUÑOZ, Begoña, 46026 VALENCIA (ES); SELVA ROLDÁN, Marta, 46026 VALENCIA (ES); SEPÚLVEDA SANCHÍS, Pilar, 46026 VALENCIA (ES); TARAZONA CAMPOS, Sonia, 46022 VALENCIA (ES); SALGUERO GARCIA, Pedro, 46022 VALENCIA (ES)
(74) Representative: Herrero & Asociados, S.L.

(57) **Abstract**

The present invention refers to an in vitro method for predicting the risk of myocardial ischemia in patients with chest pain based on the determination of the expression levels of at least one of the miRNAs from a panel of miRNA biomarkers comprising: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof, in a biological sample isolated from the patient. The present invention also refers to said panel of 40 circulating miRNAs and its use as biomarker of the risk of myocardial ischemia in patients with chest pain.

## Description

### Field of the invention

The present invention relates generally to the field of Cardiology. Particularly, the invention relates to a method for predicting the risk of cardiac ischemia in patients with chest pain. The method is based on the analysis of the expression levels of a set of 40 circulating miRNAs.

### Background of the invention

Cardiac ischemia is the leading cause of mortality and morbidity in the world. Therefore, the identification of biomarkers that predict the risk of suffering an ischemic event is one of the great challenges to improve the diagnosis and treatment of the disease. At present, the diagnosis of ischemic heart disease, once the ischemic event has occurred, is done by echocardiography, cardiac magnetic resonance imaging and the presence of changes in the electrocardiogram.

miRNAs have been involved in pivot biological processes such as drug resistance *(*Ahmad N, Haider S, Jagannathan S, Anaissie E, Driscoll JJ. MicroRNA theragnostics for the clinical management of multiple myeloma. Leukemia 2014; 28(4): 732-8*)*, cellular damage or cancer (Allegra A, Alonci A, Campo S, et al. Circulating microRNAs: new biomarkers in diagnosis, prognosis and treatment of cancer (review). Int J Oncol 2012; 41(6): 1897-912)*.* These molecules are a small class of single trans noncoding RNAs between 20-25 nucleotides that control expression of mRNAs at the post-transcriptional level by targeting the 3'untranslated region of mRNA transcripts (Ambros V. The functions of animal microRNAs. Nature 2004; 431(7006): 350-5*).* They can cleave complementary messenger mRNA targets and diminish the translation of partially complementary targets (Kim VN. MicroRNA biogenesis: coordinated cropping and dicing. Nat Rev Mol Cell Biol 2005; 6(5): 376-85). These short molecules have high specificity and many of them have a tissue specific expression (Latronico MV, Catalucci D, Condorelli G. Emerging role of microRNAs in cardiovascular biology. Circ Res 2007; 101(12): 1225-36). Due to its high stability in plasma and serum and easy detection they have been used as biomarkers in different pathologies including cardiac diseases (Gilad S, Meiri E, Yogev Y, et al. Serum microRNAs are promising novel biomarkers. PLoS One 2008; 3(9): e3148*;* Mitchell PS, Parkin RK, Kroh EM, et al. Circulating microRNAs as stable blood-based markers for cancer detection. Proc Natl Acad Sci U S A 2008; 105(30): 10513-8*;* Schulte C, Zeller T. microRNA-based diagnostics and therapy in cardiovascular disease-Summing up the facts. Cardiovasc Diagn Ther2015; 5(1): 17-36) and specific patterns of circulating miRNAs have been associated with heart failure (Tijsen AJ, Creemers EE, Moerland PD, et al. MiR423-5p as a circulating biomarker for heart failure. Circ Res 2010; 106(6): 1035-9*)* and myocardial infarction *(*Wang GK, Zhu JQ, Zhang JT, et al. Circulating microRNA: a novel potential biomarker for early diagnosis of acute myocardial infarction in humans. Eur Heart J 2010; 31(6*)).*

The present invention provides a novel predictive model based on the combination of 40 selected circulating miRNAs that can be used to stratify cardiovascular disease patients at risk of myocardial ischemia. The authors of the present invention, after a significative experimental effort, have identified a set of 40 microRNAs differentially expressed in patients that experienced transient ischemia and controls. This microRNA signature for myocardial ischemia in patients can be used for decision making of treatment regimens together with the monitorization of ischemia in patients with known coronary artery disease.

### Brief description of the drawings

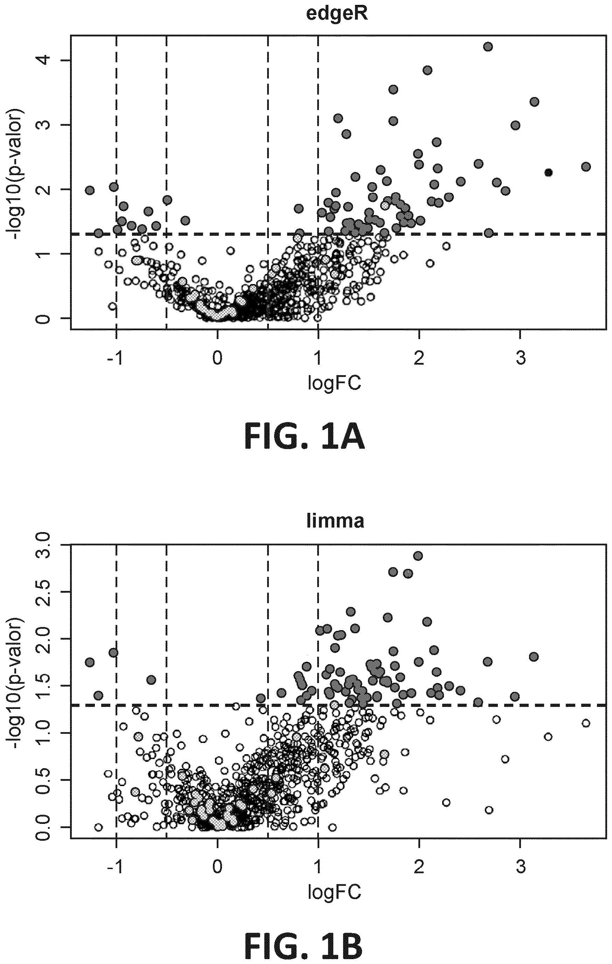

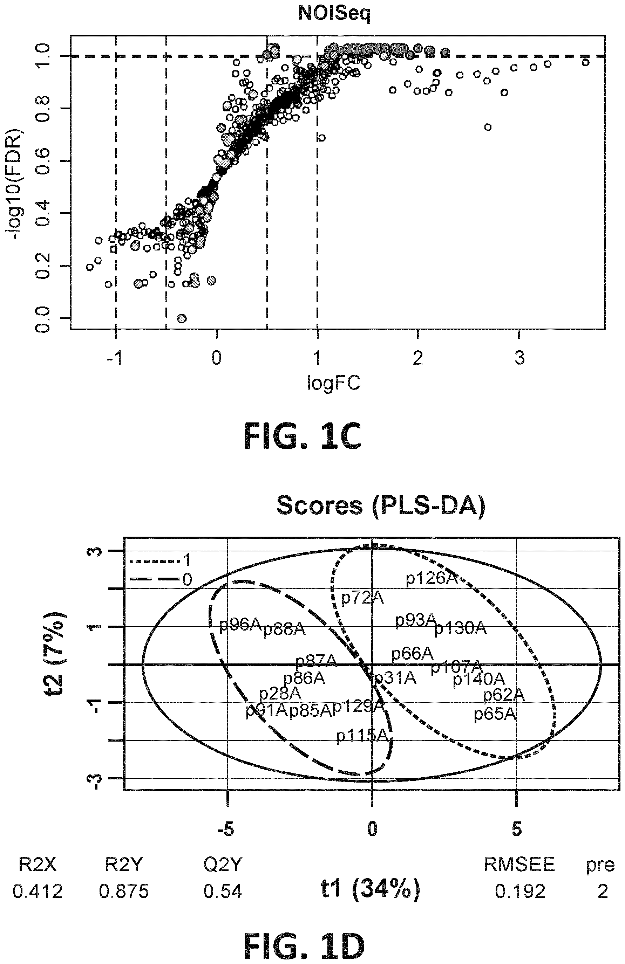

**Figure 1****.** Differential expression results with four different methods of miRNA expressed in cases (patients that experienced transient ischemia after pharmacological test) versus control group (patients that did not). (A), (B) and (C) correspond to edgeR, limma and NOISeq, respectively. All plots present log2 fold change in the X axis and -log10(p-value) in the Y axis. In red, differentially expressed miRNAs. In blue, miRNAs related to heart or cardiac damage according to literature or previous studies. Horizontal red line represents the cut-off significance value.

Vertical green lines represent different cut-off values for the log2 fold change (0.5 was finally used for miRNA selection). (D) corresponds to results for the PLS-DA model when including only the differentially expressed miRNAs returned by this method. This score plot shows how the two first components explain 87.5% of the variability of the response variable with a predictive capacity (Q²) of 0.54, and perfectly separate the two groups of patients.

### Detailed description of the invention

The present invention provides with new biomarkers of prediction of the risk of cardiac ischemia in patients with chest pain, classified as patients at risk.

In particular, the authors have developed a signature of miRNA biomarkers comprising 40 circulating miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p, that allows the prediction in vitro of the risk of myocardial ischemia in patients with chest pain.

The alteration of the expression of at least one of these miRNAs, or any combination of these circulating miRNAs, in patients with chest pain, comparing with the expression of said miRNAs in a reference sample, is indicative of risk of myocardial ischemia.

Therefore, the alteration of the expression of miRNAs thus makes it possible to distinguish patients at risk of ischemia, without the need to submit them to additional tests.

For the purposes of the present invention, myocardial ischemia is defined as a condition of reduced blood flow leading to metabolic alterations and cardiomyocyte stress that can produce ECG alterations and chest pain.

For the purposes of the present invention, chest pain refers to symptoms in patients like pressure, fullness, burning or tightness that are taken into consideration by their severity to consider them patients at risk.

This new panel of miRNA biomarkers has allowed the authors of the invention to develop an in vitro method for predicting the risk of myocardial ischemia in patients with chest pain (hereinafter "method of the invention"). This method comprises the following steps:
i) Determining the expression levels of at least one of the following miRNAs from a panel of miRNA biomarkers comprising: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof, in a biological sample isolated from the patient, and
ii) Comparing the expression levels determined in i) with the expression levels of said miRNAs in a reference sample,
wherein the alteration of the expression of these miRNAs, comparing with the expression of said miRNAs in the reference sample is indicative of risk of cardiac ischemia.

In accordance with the invention, the "reference sample", against which the expression of a panel of miRNA biomarkers is quantitatively compared, is a sample obtained from a control group of patients that do not experience transient ischemia, as assessed by cardiac magnetic resonance, after being subjected to the pharmacological stress test.

For the purposes of the invention, the determination of the miRNAs expression levels could be carried out by a Molecular Biology technique as the qPCR or any other alternative technique generally used in the state of the art.

In a preferred embodiment, the biological sample isolated from the sample is a liquid biopsy (serum, plasma, orine, blood, etc). In a more preferred embodiment, the liquid biopsy is plasma.

In a particular embodiment, the method of the invention, in step i), comprises determining the expression levels of at least one of the miRNAs from a panel of miRNAs biomarkers consisting of hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof.

In another particular embodiment, the method of the invention, in step i) comprises determining the expression levels of the miRNAs from the panel of miRNAs biomarkers consisting of the following 40 miRNAS: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p.

The present invention also refers to a panel of miRNA biomarkers (herein after "panel of the invention") comprising at least one the following miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-has3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p, or any combination thereof.

In a particular embodiment, the panel of miRNA biomarkers consists of at least one of the following miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof.

In another particular embodiment, the panel of miRNA biomarkers consists of the following 40 miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p.

In another embodiment, the present invention refers to the use of the panel of miRNA biomarkers of the invention in an in vitro method for predicting the risk of myocardial ischemia in patients with chest pain, wherein the alteration of the expression of these miRNAs, comparing with the expression of said miRNAs in a reference sample, is indicative of the risk of ischemia, wherein the panel comprises at least one of the following miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof.

In another particular embodiment, the present invention refers to the use of the panel of the invention in an in vitro method for predicting the risk of myocardial ischemia in patients with chest pain, wherein the alteration of the expression of these miRNAs, comparing with the expression of said miRNAs in a reference sample, is indicative of the risk of cardiac ischemia, wherein the panel consists on at least one the following miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p, or any combination thereof.

In another particular embodiment, the present invention refers to the use of the panel of the invention in an in vitro method for predicting the risk of myocardial ischemia in patients with chest pain, wherein the alteration of the expression of these miRNAs, comparing with the expression of said miRNAs in a reference sample, is indicative of the risk of ischemia, wherein the panel consists on the following 40 miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p.

Further, the present invention refers to the use of the panel of miRNA biomarkers of the invention as biomarker of prediction of the risk of myocardial ischemia in patients with chest pain.

Finally, the invention also refers to a kit for in vitro prediction of risk of myocardial ischemia in patients with chest pain, wherein the kit comprises means for determining quantitatively the expression level of the panel of miRNA biomarkers of the invention and an instruction for conducting the method for in vitro prediction of the risk of ischemia of the present invention, as defined in the present invention.

The following examples are illustrative only and are not intended to limit the scope of the invention.

### Examples

### Materials and Methods

The study was carried out according to the principles of the Declaration of Helsinki. The researchers assured that the privacy of the patients is guaranteed. All procedures were approved by local and national ethics committees. Written informed consent was obtained from each patient.

### Study design

Prospective observational study.

### Study population

The study population was represented by 100 consecutive patients with chest pain of possible coronary origin who underwent dipyridamole stress cardiac magnetic resonance due to medical reasons.

Exclusion criteria were a history of myocardial infarction or coronary revascularization within the last 3 months, hemodynamic instability and asthma.

### Main study parameters/endpoints

The primary endpoint is the occurrence of cardiac perfusion impaired segments after dipyridamole treatment. The presence, in cardiac magnetic resonance performed by a qualified cardiologist of the following situation:
1. NEGATIVE TEST (controls) patients without perfusion deficit at first pass perfusion images
2. POSITIVE TEST (cases) patients with perfusion deficit in at least two consecutive slices in areas without delayed enhancement.

### Patients

The study population included 100 patients who, based on clinical criteria, underwent a stress test with dipyridamole. The number of *cases* was approximately 50% (n= 50 *control* and n= 49 *cases*)*.* The period for inclusion of patients was 3 years.

The study population consisted of *control* and *cases.*

### Clinical data collection

Clinical data was collected before undergoing the stress test with dipyridamole. Data include demographics, medical history, cardiovascular risk factors, use of concomitant medication, symptoms and signs of cardiovascular disease, results of physical examination and cardiac magnetic resonance results.

### Cardiac magnetic resonance with dypiridamol stress test.

All patients were examined with a 1.5-Tsystem. Image analysis was performed using an Advantage Windows system 4.5 GE^{®} Milwakee. EEUU with Report Cart^{®}GE software, Milwakee, EEUU. The images were acquired by a phased-array body surface coil during breath holds and were electrocardiogram triggered. They were evaluated by two expert cardiologists, each with more than 5 years of experience evaluating cardiac MR images.

Left ventricular ejection fraction (LVEF) and volumes were derived from short axis cine images, papillary muscles and pericardial fat were excluded from calculations. In brief, the end-diastolic and end-systolic cine frames were identified for each slice and the endocardial and epicardial borders were manually traced. The end-diastolic and end-systolic volumes were then calculated using Simpson's true disk summation technique (i.e. sum of cavity sizes across all continuous slices) and indexed by body surface area. All images were acquired by a phased-array body surface coil during breath holds and were electrocardiogram triggered

### Cine images at rest

Left ventricular function was assessed using multiphase balance steady state free precession (b-SSFP) sequences in 2-, 3-, 4-chamber, and short-axis views (repetition time/echo time 2.8 ms/1.2 ms, flip angle 58°, matrix 256,256, field ofview 320,270 mm, slice thickness 8 mm).

### First-pass perfusion imaging

Vasodilatation was induced with dipyridamole (0.84 mg/kg body weight) delivered intravenously over 6 min. Two minutes after the end of dipyridamole infusion, 0.2 mmol/kg of gadopentate dimeglumine (Multihance ^{®}Braco Italy). Then 4 sections equally separated in the short-axis view and 2 in the 2- and 4-chamber long-axis views were acquired for first-pass perfusion imaging (b-SSFP with a saturation pulse, inversion time 125 ms, repetition time/echo time202 ms/1 ms, flip angle 50°, matrix 192-96, field of view 350-220 mm, slice thickness 8 mm).

### Cine images at stress

Once the peak myocardial enhancement was reached, multiphase balance steady state free precession (b-SSFP) sequences in one long axis and four short-axis views covering the whole left ventricular volume were used to assess left ventricular function within the peak dipyridamole-induced vasodilatation (approximately 3 min after infusion).

### Late enhancement imaging

Late enhancement imaging was performed 10 min after contrast injection in the same locations evaluated for cine images at rest using an ECG triggered inversion recovery turbo-fast gradient-echo pulse sequence with repetition time /echo time 500/1.43 ms, flip angle 10°, matrix 256x225, field of view 360 to 400 mm and inversion time of 250 to 350 ms to null normal myocardium.

A 17 segment AHA model was applied (3). 4 dipyridamole stress CMR-derived indexes were evaluated:
1. Wall motion at rest (AWM-rest): number of segments showing hypokinesis, akinesis, or dyskinesis at baseline.
2. Wall motion with dipyridamole (AWM-D): number of segments showing hypokinesis, akinesis, or dyskinesis at stress.
3. Perfusion deficit with dipyridamole: number of segments showing persistent delay (in at least 3 consecutive temporal images) in the visual analysis of enhancement pattern during the first pass of contrast through the myocardium
4. Delayed enhancement: number of segments showing enhancement in the late enhancement imaging. AWM, AWM-D, perfusion deficit, and delayed enhancement were evaluated and subsequently a NEGATIVE TEST (controls) was considered in patients without perfusion deficit at FIRST PASS perfusion images and a POSITIVE TEST (cases) in patients with perfusion deficit in at least two slices in areas without delayed enhancement.

A severe positive test was considered in patients with impaired wall motion with dypiridamol and AWM-D higher than AWM-rest.

### Plasma sample collection

Blood samples were taken before starting the stress test with dipyridamole in sodium citrate tubes. After two hours clotting, plasma was harvested and stored at the IIS La Fe.

### RNA extraction and sequencing

miRNA isolation was performed used miRNeasy kit (Qiagen Inc) according to the manufacture's instruction. 200 µl of plasma samples were thawed on ice and centrifuged for 10 min at 10,000g 4°C. The supernatant of plasma was added to QIAzol lysis reagent containing a known number of copies of cel-miR-39 as internal control. After vortex the mix, chloroform was added, and samples were incubated at room temperature for 5 min. Tubes were centrifuged for 15min at 12,000g at 4°C. 250 ul from the aqueous phase obtained after centrifugation was mixed with 375 µl of 100% ethanol. This mixture was loaded into an RNeasy minElute spin column in a 2mL collection tube and followed by centrifugation. The column was sequentially washed with Buffer RWT (700µl) and Buffer RPE (500 µl) twice. The miRNA was eluted with 25 µl of RNase-free water. In the case of samples sent for sequencing, 1000 µl of plasma samples were thawed on ice and centrifuged for 10 min at 10,000g 4°C. Next, the same protocol was used without adding the cell-miR-39.

### Reverse transcription

Reverse transcription (RT) was performed using miRCURY LNA^{™} Universal RT microRNA PCR Kit (Exiqon) following manufacturer's instructions. Briefly, 2µl of RNA was mixed with 5x Reaction Buffer (2µl), Enzyme mix (1µl) and nuclease free water (5µl) to a final volume of 10µl. The mix was incubated for 60 min at 42°C followed by transcriptase heat-inactivation by incubating 5 min at 95°C. The product was frozen down until used.

### miRNA qPCR quantification

The quantification was performed using miRCURY LNA^{™} Universal RT microRNA PCR Kit and following manufacturer's instructions. Briefly, the cDNA was diluted in a 1/80 proportion in nuclease free water. 4 µl of diluted sample was mixed with 5µl of PCR Master Mix and 1 µl of PCR primer set to a final volume of 10 µl each well. The temperature cycle program used for amplification was: 95°C for 10 min, followed by 40 cycles at 95°C for 15 s and 60°C for 1min. Real-time quantitative PCR was performed using the ViiA TM 7 Real-time PCR System (Applied Biosystems, Carlsbad, USA). Real-time monitoring of the PCR reactions was performed with the QuantStudio Real-Time PCR Software and DataAssist v3.01 (Applied Biosystems). Standard curves were made in parallel using a synthetic sequence of each miRNA. Hsa-mir-16-5p expression level was used as house-keeping control.

### miRNA sequencing

Libraries were prepared using the NEBNext^{®} Small RNA Library Prep Set for Illumina^{®} kit (ref. E7330) according to the manufacturer's protocol. Briefly, RNA was subjected to adaptor 3'and 5' ligation and first strand cDNA synthesis. After that, PCR selectively enriched those DNA fragments that had adapter molecules on both ends. Library amplification was performed by PCR using NEBNext^{®} Multiplex Oligos for Illumina (Index Primers Set 1, ref. E7335), (Index Primers Set 2, ref. E7500), (Index Primers Set 3, ref. E7710) and (Index Primers Set 4, ref. E7730). All purification steps were performed using AgenCourt AMPure XP beads (ref. A63882, Beckman Coulter). Final libraries were analyzed using Agilent Bioanalyzer (ref. 5067-4626) to estimate the quantity and check size distribution. A pool was done to perform size selection using 6% Novex TBE PAGE Gels (ref. EC6265BOX) and then final pool was quantified by qPCR using the KAPA Library Quantification Kit (ref. KK4835, KapaBiosystems) prior to amplification with Illumina's cBot. Libraries were sequenced 1 * 50+8 bp on Illumina's HiSeq2500.

### Pre-processing of miRNA-seq data

The quality of the sequencing reads provided by CRG was analyzed with FastQC software and reads were trimmed to remove the detected adaptors with cutadapt tool. Reads were mapped to the reference human transcriptome with all mature miRNA sequences (https://www.mirbase.org/ftp.shtml, file mature.fa) with Bowtie2 and miRNA expression was quantified with in-house scripts. The lowly expressed miRNAs were filtered out with the CPM method from R NOISeq package, which was also applied to detect the GC content bias in the samples that was corrected with the Conditional Quantile Normalization method (CQN).

### Differential expression analysis

Several differential expression methods were employed to search for altered miRNAs between cases and control patients: edgeR negative binomial test, limma regression models, NOISeq non-parametric test and Partial Least Squares Discriminant Analysis (PLS-DA) dimension reduction method. Non-normalized miRNA counts, and the normalization offset provided by CQN were provided to edgeR, while limma and PLS-DA were applied on the CQN normalized data, which included a log-transformation of the expression values. CQN normalized data without log-transformation was given to NOISeq. A permutation approach was used to estimate the p-value of the regression coefficients in the PLS-DA method. While a p-value cutoff of 0.05 was used to select the differentially expressed miRNAs from edgeR, limma and PLS-DA results, we used a cutoff of 0.10 for the FDR adjusted p-value from NOISeq.

The first list of altered miRNAs able to predict risk to suffer cardiac ischemia was obtained by combining different criteria:
- Being selected by at least two of the three differential expression methods (edgeR, limma and NOISeq) and presenting a logFC greater than 0.5.
- Being miRNAs related to cardiac or heart damage, presenting a logFC greater than 0.5 and having been selected (or "almost" selected, that is, a p-value very close to the cut-off value) by at least one of the three previous methods.
- Being selected by the multivariate PLS-DA method.

We next refined the statistically significant miRNA to a group of miRNAs with biological significance to the pathophysiological mechanism of myocardial ischemia. The final list of altered miRNAs able to predict risk to suffer cardiac ischemia was obtained attending the following criteria: i) miRNA previously reported in cardiac tissue, ii) miRNA involved in ischemia or apoptotic processes, iii) miRNA involved in fibrosis, iv) miRNA involved in heart development.

### Enrichment analysis

Two types of enrichment analysis were applied on the selected differentially expressed miRNAs. First, an enrichment analysis of target genes with the Fisher's Exact Test. The target genes of the quantified miRNAs were obtained from databases in multiMiR R package (version 2.3.0) and applied a Fisher's Exact Test for each target gene. Second, a functional enrichment analysis of the enriched target genes of DE miRNAs. For that, we used Gene Ontology functional terms and applied again the Fisher's Exact Test.

### Results

Patients were classified as controls (n=10) or cases (n=10) as a result of the number of segments affected during cardiac resonance and the diagnosis of transient ischemia during Dipyiriydamole test (Menadas et al., 2016). Plasma samples were harvested before and after the functional test. Demographic data are shown in Table 1.

**Table 1. Clinical characteristics of the study population**

| | **Control (n=10) Mean±SD** | **Cases (n=10) Mean±SD** | **Statistical significance** |
|---|---|---|---|
| Age (years) | 67±14 | 71±.6 | 0.353 |
| Height (cm) | 164±12 | 167±7 | 0.508 |
| Weight (kg) | 79±19 | 77±11 | 0.779 |
| BSA (m²) | 1.9±0.3 | 1.9±0.1 | 0.792 |
| BMI (kg/m²) | 28.9±3.4 | 27.3±3.4 | 0.378 |
| Gender, male, n (%) | 4 (40) | 9 (82) | 0.049 |
| Hypertension, n (%) | 8 (80) | 7 (70) | 0.606 |
| Diabetes mellitus, n (%) | 4(40) | 3 (30) | 0.639 |
| Dyslipidemia, n (%) | 3 (30) | 8(80) | 0.025 |
| Smoking, n (%) | 2 (20) | 7 (70) | 0.025 |
| Previous CVD | 3 (30) | 9 (90) | 0.006 |

| **Cardioprotective drugs** | | | |
|---|---|---|---|
| β blockers | 5 (50) | 7 (70) | 0.284 |
| ARAII, ACEI | 7 (70) | 4 (40) | 0.371 |
| Cₐ²⁺ antagonist | 3 (30) | 3 (30) | 1.000 |
| statins | 3 (30) | 9 (90) | 0.006 |
| antiplatelet agents | 5 (50) | 8 (80) | 0.160 |

| | | | |
|---|---|---|---|
| *BSA: Body surface area, BMI: Body mass index, CVD: Cardiovascular disease. Student's* *t-test was used for normally distributed variables, and the Mann Whitney test for non-normally distributed quantitative variables. Qualitative variables were compared using the* *Chi-square test.* | | | |

Cardiac function was monitored in these patients during the stress test with dipyridamole. Cardiac magnetic resonance (CMR) parameters are described in Table 2.

**Table 2. CMR characteristics of the study population.**

| | **Control (n=10)** | **Case (n=10)** | **Statistical significance** |
|---|---|---|---|
| **CMR parameters** | **Mean±SD** | **Mean±SD** | |
| LVEDV (ml/m²) | 119.3±59 | 139.4±35 | 0.426 |
| LVESV (ml/m²) | 44.3±30 | 47.9±25 | 0.806 |
| LVEF (%) | 61.6±18 | 68.8±11 | 0.456 |
| RVEDV (ml/m²) | 99.8±38 | 121.1±39 | 0.306 |
| RVESV (ml/m²) | 44.3±22 | 45.3±18 | 0.711 |
| RVEF (%) | 51.6±17 | 62.1±7 | 0.159 |
| N° hypoperfused segments after dipyridamole | 0 | 5.2±1.6 | 0.000 |

| | | | |
|---|---|---|---|
| *LVEDV: left ventricular end-diastolic volume, LVESV: left ventricular end-systolic volume,* *LVEF: left ventricular ejection fraction; RVEDV: right ventricular end-diastolic volume,* *RVESV: right ventricular end-systolic volume, RVEF: right ventricular ejection fraction.* | | | |

miRNA levels in plasma samples were determined by miRNAseq (see methods). A miRNAseq analysis using Illumina platform was performed and 812 miRNAs were detected and normalized by relative abundance and for GC content bias.

By combining the results of four differential expression methods (edgeR, limma, NOISeq and PLS-DA), the most differentially expressed miRNAs (89) between cases and controls were selected. Figure 1 shows the results of the four differential expression methods.

The final PLS-DA model, after feature selection, achieved a cross-validated accuracy of 100% (100% sensitivity and 100% sensibility), and was validated with resampling approaches, which proved the absence of overfitting. It selected 15 miRs (coefficient p-value<0.05) as the most important predictors for discriminating both groups. The edgeR negative binomial test found significant differences between both groups in 80 miRs (p-value<0.05), 11 of them also present in the 15 miRs selected by PLS-DA. The limma method also selected 80 miRs (p-value<0.05). Ten of them were included in the 15 selected by PLS-DA.

The list of 89 DE miRNAs was refined to select the most significant ones or the ones with more relevance in cardiac diseases. Thus, the list of miRNAs was refined attending the following criteria: i) miRNA previously reported in cardiac tissue, ii) miRNA involved in ischemia or apoptotic processes, iii) miRNA involved in fibrosis, iv) miRNA involved in heart development. After this screening, 40 miRNA were selected (Table 3) and their putative target genes and biological processes were identified using in silico analysis.

**Table 3. miRNA signature for predicting cardiac ischemia.**

| **miRNA** | **logFC** | **PVAL_edgeR** | **PVAL_limma** | **PVAL_plsda** | **FDR_noiseq** |
|---|---|---|---|---|---|
| **hsa-miR-10b-5p** | 1.444 | 0.038 | 0.153 | 0.275 | 0.098 |
| **hsa-miR-1199-5p** | 1.277 | 0.001 | 0.036 | 0.201 | 0.095 |
| **hsa-miR-1249-3p** | 1.279 | 0.033 | 0.083 | 0.297 | 0.094 |
| **hsa-miR-1253** | 2.582 | 0.004 | 0.046 | 0.332 | 0.117 |
| **hsa-miR-125b-2-3p** | 1.816 | 0.018 | 0.025 | 0.253 | 0.097 |
| **hsa-miR-1290** | 1.397 | 0.035 | 0.146 | 0.198 | 0.093 |
| **hsa-miR-1306-5p** | 1.368 | 0.006 | 0.008 | 0.015 | 0.104 |
| **hsa-miR-138-5p** | 1.618 | 0.005 | 0.022 | 0.095 | 0.093 |
| **hsa-miR-145-3p** | 1.117 | 0.027 | 0.02 | 0.124 | 0.096 |
| **hsa-miR-216a-5p** | 0.807 | 0.02 | 0.024 | 0.176 | 0.153 |
| **hsa-miR-218-5p** | 1.367 | 0.046 | 0.176 | 0.803 | 0.099 |
| **hsa-miR-3164** | 1.44 | 0.042 | 0.036 | 0.257 | 0.094 |
| **hsa-miR-328-5p** | 1.745 | 0 | 0.013 | 0.09 | 0.105 |
| **hsa-miR-329-3p** | -1.262 | 0.01 | 0.017 | 0.044 | 0.636 |
| **hsa-miR-365a-5p** | 2.406 | 0.008 | 0.035 | 0.323 | 0.118 |
| **hsa-miR-3675-3p** | 1.442 | 0.047 | 0.059 | 0.205 | 0.098 |
| **hsa-miR-410-3p** | -1.024 | 0.009 | 0.014 | 0.02 | 0.542 |
| **hsa-miR-4488** | 1.742 | 0.001 | 0.002 | 0.015 | 0.134 |
| **hsa-miR-4786-5p** | 1.759 | 0.016 | 0.032 | 0.197 | 0.096 |
| **hsa-miR-493-5p** | -1.179 | 0.049 | 0.04 | 0.159 | 0.599 |
| **hsa-miR-5006-5p** | 1.68 | 0.007 | 0.028 | 0.097 | 0.093 |
| **hsa-miR-615-3p** | 1.842 | 0.033 | 0.039 | 0.167 | 0.093 |
| **hsa-miR-657** | 1.819 | 0.026 | 0.064 | 0.293 | 0.092 |
| **hsa-miR-671-5p** | 1.889 | 0.026 | 0.002 | 0.072 | 0.128 |
| **hsa-miR-6865-3p** | 2.296 | 0.013 | 0.031 | 0.242 | 0.138 |
| **hsa-miR-7106-3p** | 2.191 | 0.017 | 0.039 | 0.268 | 0.115 |
| **hsa-miR-9-5p** | 2.947 | 0.001 | 0.041 | 0.236 | 0.111 |
| **hsa-miR-1260a** | 1.582 | 0.107 | 0.04 | 0.268 | 0.094 |
| **hsa-miR-214-3p** | 1.159 | 0.207 | 0.049 | 0.266 | 0.099 |
| **hsa-miR-6889-5p** | 1.52 | 0.189 | 0.018 | 0.097 | 0.093 |
| **hsa-miR-8485** | 1.598 | 0.056 | 0.023 | 0.22 | 0.093 |
| **hsa-miR-877-3p** | 1.217 | 0.101 | 0.022 | 0.153 | 0.095 |
| **hsa-miR-1-3p** | 1.657 | 0.018 | 0.166 | 0.385 | 0.1 |
| **hsa-let-7c-5p** | 0.787 | 0.058 | 0.108 | 0.193 | 0.103 |
| **hsa-miR-4732-3p** | 0.582 | 0.166 | 0.296 | 0.184 | 0.095 |
| **hsa-let-7a-5p** | 0.538 | 0.206 | 0.435 | 0.412 | 0.093 |
| **hsa-miR-221-3p** | -0.357 | 0.483 | 0.132 | 0.049 | 0.534 |
| **hsa-miR-342-3p** | 0.676 | 0.109 | 0.095 | 0.047 | 0.124 |
| **hsa-miR-494-3p** | -0.496 | 0.015 | 0.187 | 0.021 | 0.419 |
| **hsa-miR-92b-3p** | 1.154 | 0.02 | 0.162 | 0.039 | 0.114 |

The target genes enrichment analysis returned 470 enriched targets (p-value < 0.01). The top 20 most significant ones are displayed in Table 4. A functional enrichment analysis was performed on the enriched target genes with the Gene Ontology functional terms. We found 108 enriched GO terms. Table 5 shows 20 of the most significant ones.

**Table 4. Top 20 most significant enriched target genes on the 40 selected altered miRNAs.**

| | **target_ensembl** | **annotTest** | **test** | **annotNotTest** | **notTest** | **pval** |
|---|---|---|---|---|---|---|
| TMEM201 | ENSG00000188807 | 9 | 40 | 24 | 772 | 0.0000111 |
| PRKAG1 | ENSG00000181929 | 9 | 40 | 25 | 772 | 0.0000145 |
| ELP3 | ENSG00000134014 | 6 | 40 | 9 | 772 | 0.0000353 |
| WAPL | ENSG00000062650 | 10 | 40 | 37 | 772 | 0.0000368 |
| NUDT9 | ENSG00000170502 | 5 | 40 | 5 | 772 | 0.0000475 |
| PAX3 | ENSG00000135903 | 7 | 40 | 16 | 772 | 0.0000565 |
| NUDT16 | ENSG00000198585 | 7 | 40 | 17 | 772 | 0.0000769 |
| BNIP3L | ENSG00000104765 | 9 | 40 | 34 | 772 | 0.0001128 |
| OIP5 | ENSG00000104147 | 7 | 40 | 19 | 772 | 0.0001357 |
| GEM | ENSG00000164949 | 7 | 40 | 19 | 772 | 0.0001357 |
| STARD4 | ENSG00000164211 | 10 | 40 | 45 | 772 | 0.0001563 |
| TCTE1 | ENSG00000146221 | 4 | 40 | 3 | 772 | 0.0001596 |
| VPS4A | ENSG00000132612 | 8 | 40 | 28 | 772 | 0.0001858 |
| RPL8 | ENSG00000161016 | 8 | 40 | 28 | 772 | 0.0001858 |
| ACAT1 | ENSG00000075239 | 5 | 40 | 8 | 772 | 0.0002171 |
| TTLL4 | ENSG00000135912 | 7 | 40 | 21 | 772 | 0.0002270 |
| P4HA2 | ENSG00000072682 | 6 | 40 | 14 | 772 | 0.0002271 |
| ZNF780A | ENSG00000197782 | 8 | 40 | 29 | 772 | 0.0002286 |
| ATF4 | ENSG00000128272 | 8 | 40 | 29 | 772 | 0.0002286 |
| DUSP10 | ENSG00000143507 | 9 | 40 | 38 | 772 | 0.0002358 |

**Table 5. Gene ontology (GO) biological processes associated with differentially expressed miRNAs.**

| **GO_id** | **Term** | **p-value** |
|---|---|---|
| GO:0008285 | negative regulation of cell population proliferation | 9.67E-05 |
| GO:0034391 | regulation of smooth muscle cell apoptotic process | 6.61E-04 |
| GO:0032745 | positive regulation of interleukin-21 production | 6.61E-04 |
| GO:0005925 | focal adhesion | 1.29E-03 |
| GO:0043065 | positive regulation of apoptotic process | 1.39E-03 |
| GO:2001045 | negative regulation of integrin-mediated signaling pathway | 1.95E-03 |
| GO:0060070 | canonical Wnt signaling pathway | 2.92E-03 |
| GO:1901509 | regulation of endothelial tube morphogenesis | 3.83E-03 |
| GO:0044262 | cellular carbohydrate metabolic process | 3.83E-03 |
| GO:0010575 | positive regulation of vascular endothelial growth factor production | 5.43E-03 |
| GO:0043066 | negative regulation of apoptotic process | 5.73E-03 |
| GO:0010718 | positive regulation of epithelial to mesenchymal transition | 5.79E-03 |
| GO:1903543 | positive regulation of exosomal secretion | 6.12E-03 |
| GO:0006915 | apoptotic process | 7.63E-03 |
| GO:0045766 | positive regulation of angiogenesis | 7.88E-03 |
| GO:0042981 | regulation of apoptotic process | 8.10E-03 |
| GO:0060071 | Wnt signaling pathway | 8.31E-03 |
| GO:0043619 | regulation of transcription from RNA polymerase II promoter in response to oxidative stress | 9.26E-03 |
| GO:0000963 | mitochondrial RNA processing | 9.26E-03 |
| GO:2000391 | positive regulation of neutrophil extravasation | 9.26E-03 |

To note that BNIP3L related with apoptotic processes were differentially expressed between control and cases (Liu, W., Wang, X., Mei, Z., Gong, J., Gao, X., Zhao, Y., & Qian, L. (2017). BNIP3L promotes cardiac fibrosis in cardiac fibroblasts through [Ca 2+] i-TGF-β-Smad2/3 pathway. Scientific reports, 7(1), 1-13*;* Ouyang, S., Chen, W., Zeng, G., Lei, C., Tian, G., Zhu, M. & Yang, M. (2020). MicroRNA-183-3p up-regulated by vagus nerve stimulation mitigates chronic systolic heart failure via the reduction of BNIP3L-mediated autophagy. Gene, 726, 144136). Pax3 and ELP3 that are implicated in developmental and disease processes were also identified (Boudjadi, S., Chatterjee, B., Sun, W., Vemu, P., & Barr, F. G. (2018). The expression and function of PAX3 in development and disease. Gene, 666, 145-157; Coles, S., Giamberardino, S., Haynes, C., She, R., Gui, H., Kraus, W. E., ... & Shah, S. H. (2020). Genetic Variation Associated With Favorable Exercise Response in Patients With Systolic Heart Failure: A Hf-action Trial Substudy. Circulation, 142(Suppl_3), A16003-A16003)*.* Many of the GO biological processes identified, were involved with the regulation of apoptotic and angiogenesis processes. To note that both mechanisms are directly related to ischemic process. Curiously, the Wnt signaling pathway that is involved in heart development processes (Pahnke, A., Conant, G., Huyer, L. D., Zhao, Y., Feric, N., & Radisic, M. (2016). The role of Wnt regulation in heart development, cardiac repair and disease: A tissue engineering perspective. Biochemical and biophysical research communications, 473(3), 698-703*)* was also identified.

## Claims

1. An in vitro method for predicting the risk of myocardial ischemia in patients with chest pain comprising:
i. Determining the expression level of at least one of the circulating miRNA from a panel of miRNA biomarkers comprising: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof, in a biological sample isolated from the patient, and
ii. Comparing the expression levels determined in i) with the expression levels of said miRNAs in a reference sample,
wherein the alteration of the expression of these miRNAs, comparing with the expression of said miRNAs in the reference sample, is indicative of the risk of cardiac ischemia.

2. The method, according to claim 1, wherein step i) comprises determining the expression level of at least one of the circulating miRNA from a panel of miRNA biomarkers consisting of hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof, in a biological sample isolated from the patient.

3. Method according to claim 2, wherein step i) comprises determining the expression levels of the miRNAs from the panel of miRNAs biomarkers consisting of the following 40 miRNAS: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p.

4. Method according to any one of claims 1 to 3, wherein the biological sample is plasma.

5. A panel of miRNA biomarkers comprising at least one of the following circulating miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p, or any combination thereof.

6. A panel of miRNA biomarkers according to claim 5, consisting of at least one of the following circulating miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p, or any combination thereof.

7. A panel of miRNA biomarkers, according to claim 6, consisting of the following circulating miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p and hsa-miR-92b-3p.

8. A panel of circulating miRNA biomarkers comprising at least one of the following circulating miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p or any combination thereof, for use in an in vitro method for predicting the risk of myocardial ischemia in patients with chest pain, wherein the alteration of the expression of these miRNAs, comparing with the expression of said miRNAs in a reference sample, is indicative of the risk of ischemia.

9. The panel of circulating miRNA biomarkers for use, according to claim 8, consisting of at least one of the following circulating miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p, or any combination thereof.

10. The panel of circulating miRNA biomarkers for use, according to claim 9, consisting on the following circulating miRNAs: hsa-miR-10b-5p, hsa-miR-1199-5p, hsa-miR-1249-3p, hsa-miR-1253, hsa-miR-125b-2-3p, hsa-miR-1290, hsa-miR-1306-5p, hsa-miR-138-5p, hsa-miR-145-3p, hsa-miR-216a-5p, hsa-miR-218-5p, hsa-miR-3164, hsa-miR-328-5p, hsa-miR-329-3p, hsa-miR-365a-5p, hsa-miR-3675-3p, hsa-miR-410-3p, hsa-miR-4488, hsa-miR-4786-5p, hsa-miR-493-5p, hsa-miR-5006-5p, hsa-miR-615-3p, hsa-miR-657, hsa-miR-671-5p, hsa-miR-6865-3p, hsa-miR-7106-3p, hsa-miR-9-5p, hsa-miR-1260a, hsa-miR-214-3p, hsa-miR-6889-5p, hsa-miR-8485, hsa-miR-877-3p, hsa-miR-1-3p, hsa-let-7c-5p, hsa-miR-4732-3p, hsa-let-7a-5p, hsa-miR-221-3p, hsa-miR-342-3p, hsa-miR-494-3p, hsa-miR-92b-3p.

11. Use of the panel of miRNA biomarkers as defined in claims 5-7 as biomarker of prediction of the risk of myocardial ischemia in patients with chest pain.

12. A kit for in vitro prediction of the risk of myocardial ischemia in patients with chest pain, **characterized in that** it comprises means for determining quantitatively the expression level of the panel of miRNA biomarkers as defined in any of claims 5-7 and an instruction for conducting the method for in vitro prediction of the risk of ischemia as defined in any of claims 1-4.
